# EUROPEAN PATENT APPLICATION

(11) **EP 3 964 219 A1**
(43) Date of publication of application: **09.03.2022**
(21) Application number: 20798808.0
(22) Date of filing: 27.04.2020
(51) Int. Cl.: A61K 35/19, A61K 35/28, A61K 45/06, A61P 31/00

(54) **PHARMACEUTICAL COMPOSITION FOR TREATING SEPSIS OR SYSTEMIC INFLAMMATORY RESPONSE SYNDROME, COMPRISING ISOLATED MITOCHONDRIA AS ACTIVE INGREDIENT**

(30) Priority: 29.04.2019 KR 20190050017
(71) Applicant: Paean Biotechnology Inc., Daejeon 34013 (KR)
(72) Inventor: HAN, Kyuboem, Daejeon 34028 (KR); KIM, Chun-Hyung, Sejong 30130 (KR); YU, Shin-Hye, Daejeon 34018 (KR); LEE, Seo-Eun, Daejeon 34036 (KR); LIM, Sang-Min, Incheon 21387 (KR); JUNG, Hahnsun, Gunpo-si Gyeonggi-do 15824 (KR); NA, Kwangmin, Gwangmyeong-si Gyeonggi-do 14309 (KR); HAN, Yoon Mi, Seoul 02043 (KR); SON, Jun Young, Seoul 01126 (KR); HA, Jong-Cheon, Daejeon 34048 (KR); JUNG, Kyunghee, Sejong 30016 (KR); KANG, Young-Cheol, Wonju-si Gangwon-do 26463 (KR); LEE, Eun-Seo, Okcheon-gun Chungcheongbuk-do 29001 (KR); KIM, Mi Jin, Daejeon 34028 (KR); CHOI, Yong-Soo, Gunpo-si Gyeonggi-do 15888 (KR); HWANG, Jung Uk, Sejong 30082 (KR); LEE, Min Ji, Yongin-si Gyeonggi-do 16865 (KR); KIM, Kyuseok, Seoul 06270 (KR); CHUNG, Tae Nyoung, Seoul 06521 (KR)
(74) Representative: HGF
(86) International application number: PCT/KR2020/005537
(87) International publication number: WO 2020/222483

(57) **Abstract**

The present invention relates to a pharmaceutical composition for the treatment of sepsis or systemic inflammatory response syndrome (SIRS) comprising mitochondria as effective ingredient. When activated macrophages and monocytes are treated with mitochondria which are effective ingredient of the pharmaceutical composition of the present invention, expression of IL-1β, TNF-α and IL-6 which are pro-inflammatory cytokines can be restored to normal levels. Furthermore, when the pharmaceutical composition of the present invention is administered to a subject suffering from sepsis, the survival rate of the subject can be remarkably increased. Therefore, the pharmaceutical composition according to the present invention can be useful for the treatment of sepsis.

<110>     Paean Biotechnology Inc.

<120>     PHARMACEUTICAL COMPOSITION FOR TREATING SEPSIS OR SYSTEMIC
          INFLAMMATORY RESPONSE SYNDROME COMPRISING ISOLATED MITOCHONDRIA
          AS EFFECTIVE INGREDIENT

<130>     SPO20-006_PCT_PBT

<150>     KR 10-2019-0050017
<151>     2019-04-29

<160>     12

<170>     KoPatentIn 3.0

<210>     1
<211>     20
<212>     DNA
<213>     Artificial Sequence

<220>
<223>     Human IL-6-S


<400>     1
ccacacagac agccactcac                                                   20


<210>     2
<211>     20
<212>     DNA
<213>     Artificial Sequence

<220>
<223>     Human IL-6-AS


<400>     2
tttcaccagg caagtctcct                                                   20


<210>     3
<211>     21
<212>     DNA
<213>     Artificial Sequence

<220>
<223>     Human 18S-S


<400>     3
ctcccacttg gataactgtg g                                                 21


<210>     4
<211>     20
<212>     DNA
<213>     Artificial Sequence

<220>
<223>     Human 18S-AS

<400>    4
gaccgggttg gttttgatct                                                    20


<210>    5
<211>    20
<212>    DNA
<213>    Artificial Sequence

<220>
<223>    TNF-alpha-S


<400>    5
tctcatcagt tctatggccc                                                    20


<210>    6
<211>    20
<212>    DNA
<213>    Artificial Sequence

<220>
<223>    TNF-alpha-AS


<400>    6
gggagtagac aaggtacaac                                                    20


<210>    7
<211>    22
<212>    DNA
<213>    Artificial Sequence

<220>
<223>    IL-1beta-F


<400>    7
aacctgctgg tgtgtgacgt tc                                                 22


<210>    8
<211>    22
<212>    DNA
<213>    Artificial Sequence

<220>
<223>    IL-1beta-R


<400>    8
   cagcacgagg cttttttgtt gt                                                  22


<210>    9
<211>    21
<212>    DNA
<213>    Artificial Sequence

<220>
<223>    IL-6-AS

<400>    9
ctaggtttgc cgagtagatc t                                                21

<210>    10
<211>    24
<212>    DNA
<213>    Artificial Sequence

<220>
<223>    IL-6-S

<400>    10
ccaaactgga tataatcagg aaat                                             24

<210>    11
<211>    20
<212>    DNA
<213>    Artificial Sequence

<220>
<223>    GAPDH-S

<400>    11
ggtgaaggtc ggtgtgaacg                                                  20

<210>    12
<211>    20
<212>    DNA
<213>    Artificial Sequence

<220>
<223>    GAPDH-AS

<400>    12
ctcgctcctg gaagatggtg                                                  20

## Description

### Technical Field

The present invention relates to a pharmaceutical composition for treating sepsis or systemic inflammatory response syndrome comprising mitochondria as an effective ingredient.

### Background Art

"Systemic inflammatory response syndrome" refers to a condition in which a severe inflammatory reaction occurs throughout the body. Systemic inflammatory response syndrome (SIRS) is defined as a case where a patient exhibits two or more symptoms among hyperthermia in which the body temperature rises above 38°C or hypothermia in which the body temperature falls below 36°C, a respiratory rate more than 24 times per minute (tachypnea), a heart rate more than 90 times per minute (tachycardia), or an increase or significant decrease in the number of white blood cells on blood tests. Systemic inflammatory response may be caused by trauma, burns or infections.

Sepsis refers to a disease in which severe systemic inflammatory responses are caused by infection with microorganism (including virus). Sepsis is defined as a case where a patient exhibits two or more symptoms among hyperthermia in which body temperature is higher than 38°C, hypothermia in which body temperature is lower than 36°C, a respiratory rate greater than 24 breaths/min (tachypnea), a heart rate greater than 90 beats/min (tachycardia), and an increase or decrease in blood white blood cell count. Sepsis is a disease with 30% mortality rate and 30 million patients occurring each year all over the world.

Sepsis is treated by finding an infection site in the body which causes sepsis, through physical examination, blood test, and imaging test, and then prescribing appropriate antibiotics. In addition, medication, including antibiotics, for maintaining various homeostasis mechanisms in the body, and supportive therapy for overcoming each organ dysfunction are applied in combination for treatment of sepsis. In order to find out a causative microorganism of sepsis, however, the patient's blood should be collected and cultured to check the microorganism, which takes at least 3 to 5 days. However, patients may die within a short time after the onset of sepsis, which causes difficulty in treating sepsis. There is no fundamental cure for sepsis to date.

Various anti-inflammatory substances and immunomodulators have been tried as therapeutic agents to suppress the hyperinflammatory response of sepsis. Specifically, attempts have been made to treat sepsis using immunomodulators such as corticosteroids, anti-endotoxin antibodies, TNF antagonists, and IL-1 receptor antagonists, but so far, no satisfactory results have been obtained to improve the prognosis for sepsis.

The reasons why it is difficult to treat sepsis using anti-inflammatory substances and immunomodulators are as follows: i) Both the pro-inflammatory state and the anti-inflammatory state appear in the immune system of sepsis patients, which requires anti-inflammatory substances or immunomodulators to be prescribed taking into account the immune system status of each sepsis patient; ii) The progress of infection varies and, thus, the immune system status is different depending on the site of infection; iii) The effect of anti-inflammatory substances or immunomodulators is different between infection by Gram-negative bacteria and Gram-positive bacteria *(*The Journal of the Korean Medical Association, 49(7):634, 2006).

Meanwhile, mitochondria are a cellular organelles in eukaryotic cells involved in the synthesis and regulation of adenosine triphosphate (ATP) as an intracellular energy source. Mitochondria are associated with various metabolic pathways *in vivo,* such as cell signaling, cell differentiation, apoptosis, as well as the control of cell cycle and cell growth.

### Disclosure of Invention

### Technical Problem

Although research has been conducted to treat sepsis, developed drugs have insufficient therapeutic effects or have adverse effects. Thus, there is a need for development of a safe and effective therapeutic agent for sepsis. Accordingly, an object of the present invention is to provide a pharmaceutical composition for treating sepsis.

### Solution to Problem

In order to solve the above technical problem, there is provided a pharmaceutical composition for treating sepsis or systemic inflammatory response syndrome (SIRS) comprising mitochondria as an effective ingredient in an aspect of the present invention.

In another aspect of the present invention, a method is provided for treating sepsis or systemic inflammatory response syndrome (SIRS), comprising a step of administering the pharmaceutical composition to a subject.

### Advantageous Effects of Invention

When activated macrophage cells are treated with mitochondria which are an effective ingredient of a pharmaceutical composition of the present invention, expression of IL-1 β, TNF-α and IL-6 (pro-inflammatory cytokines) can be restored to normal levels. In addition, when a pharmaceutical composition of the present invention is administered to a subject to which sepsis has been artificially induced, a survival rate of the subject can be remarkably increased. Therefore, a pharmaceutical composition from the present invention can be useful in treating sepsis.

### Brief Description of Drawings

Fig. 1 illustrates TNF-α secretion from mouse Raw 264.7 cells treated with lipopolysaccharide (LPS) which were treated with mitochondria at respective concentrations.
Fig. 2 illustrates IL-6 secretion from mouse Raw 264.7 cells treated with LPS which were treated with mitochondria at respective concentrations.
Fig. 3 illustrates the ability to inhibit IL-6 mRNA expression in human THP-1 cells activated with LPS which were treated with mitochondria derived from various types of cells.
Fig. 4 illustrates the ability to inhibit IL-6 protein expression in human THP-1 cells activated with LPS which were treated with mitochondria derived from various types of cells.
Fig. 5 illustrates the ability to inhibit TNF-α, IL-Iβ and IL-6 mRNA expression in mouse RAW 264.7 cells activated with LPS which were treated with mitochondria derived from various types of cells.
Fig. 6 shows the increase in survival rate of the LPS-induced sepsis mouse model after the treatment with mitochondria cultured and extracted from mesenchymal stem cells derived from human umbilical cord (UC-MSC).
Fig. 7 compares 14-day rat survival curves obtained by subjecting CLP (Cecal Ligation and Puncture) induced sepsis rat model to the treatment with physiological saline, an antibiotic alone, mitochondria alone, or a combination of mitochondria and an antibiotic
Fig. 8 compares 14-day rat survival curves obtained by subjecting a CLP-induced sepsis rat model treated with an antibiotic immediately after induction of sepsis to the treatment with physiological saline, or a combination of mitochondria and an antibiotic.
Fig. 9 compares ATP activity of mitochondria derived from cryopreserved L6 cell and mitochondria derived from cultured L6 cell where L6 cells are a myoblast cell line derived from rat skeletal muscle.
Fig. 10 compares membrane potential of mitochondria derived from cryopreserved L6 cell and mitochondria derived from cultured L6 cell where L6 cells are a myoblast cell line derived from rat skeletal muscle.
Fig. 11 illustrates the number of mitochondria in a solution containing mitochondria at a concentration of 1 µg/mL measured by using the particle counter (Multisizer 4e, Beckman Coulter).
Fig. 12 illustrates the number of mitochondria in a solution containing mitochondria at a concentration of 2.5 µg/mL measured by using the particle counter.
Fig. 13 illustrates the number of mitochondria in a solution containing mitochondria at a concentration of 5 µg/mL measured by using the particle counter.

### Mode for Carrying out the Invention

Hereinafter, the present invention will be described in detail.

### Treatment for sepsis or SIRS comprising mitochondria as an effective ingredient

An aspect of the present invention is to provide a pharmaceutical composition for treating sepsis or systemic inflammatory response syndrome (SIRS) comprising mitochondria as an effective ingredient.

As used herein, the term "systemic inflammatory response syndrome" refers to a condition in which a severe inflammatory reaction occurs throughout the body. Systemic inflammatory response syndrome (SIRS) is defined as a case where two or more of the symptoms among hyperthermia in which the body temperature rises above 38°C or hypothermia that falls below 36°C, a respiratory rate more than 24 times per minute (tachypnea), a heart rate more than 90 times per minute (tachycardia), and an increase or significant decrease in the number of white blood cells on blood tests. Systemic inflammatory reactions may be caused by trauma or burns. In particular, when the systemic inflammatory response syndrome is caused by infections, it is called "sepsis".

As used herein, the term "sepsis" generally refers to a disease in which severe systemic inflammatory responses are caused by infection with microorganism. In particular, sepsis may cause abnormalities in the immune system of a subject due to the infection, which may lead to severe organ damages in the subject. Specifically, according to a study on pathophysiology of sepsis, immune cells such as neutrophils, macrophage cells, and monocytes are activated in sepsis after infection with a microorganism. Due to activation of such immune cells, secretion of inflammatory cytokines such as IL-1, IL-6, IL-8, and TNF-α are increased, and NF-κB, a transcription factor present in cells, is activated, leading to systemic inflammatory responses. If a proper treatment is not carried out in time, a shock state or death may be resulted.

In this case, sepsis may be caused by infection by microorganisms, and the microorganisms may be bacteria, fungi, or viruses. The microorganisms that can cause sepsis may be, but are not limited to, Staphylococcus species belonging to Gram-positive bacteria (Streptococcus species) and Enterococcus species. Specifically, the microorganism that causes sepsis may be Streptococcus pneumoniae or Staphylococcus aureus. In addition, the microorganism may be the Gram-negative bacteria such as the genus Klebsiella (Klebsiella species), Pseudomonas (Pseudomonas species), Enterobacter (Enterobacter species), but are not limited thereto. Specifically, the microorganisms that cause sepsis may be Escherichia coli, Vibrio vulnificus, or Hemophilus influenzae. In particular, sepsis can be caused by lipopolysaccharides present in microorganisms.

In addition, sepsis can be caused by infection by a virus, and the virus may be either Influenza virus, Adenovirus, Herpes simplex virus, Measles virus, Lentivirus, Retrovirus, Cytomegalovirus, Baculovirus, Reovirus, Adeno-associated virus, Myxoma virus, Vesicular stomatitis virus, Poliovirus, Newcastle disease virus, Parvovirus, Coxsackie virus, Seneca virus, Vaccinia virus, Corona virus or Poxvirus. In this case, the virus may be wild-type or mutant. The pharmaceutical composition containing mitochondria of the present invention as an effective ingredient can effectively control the systemic inflammatory response, and can be effectively used for sepsis as well as septic shock.

Unless stated otherwise in the present specification, the term "effective ingredient" refers to an ingredient that exhibits activity alone or in combination with an adjuvant (carrier) that is not active on its own.

In this case, the mitochondria may be isolated mitochondria. The mitochondria may be obtained either from eukaryotes or from mammals, preferably humans. Specifically, the mitochondria may be isolated from tissue, blood, or cells. For example, the mitochondria may be obtained from platelets, somatic cells, germ cells or stem cells. In addition, the mitochondria may be normal mitochondria obtained from cells in which the biological activity of mitochondria is normal. In addition, the mitochondria may be cultured in vitro.

In addition, the mitochondria may be of autologous, allogenic, or xenogenic origin. Specifically, autologous mitochondria refer to mitochondria obtained from tissues or cells of the same individual. Also, allogenic mitochondria refer to mitochondria obtained from an individual that belongs to the same species as the individual and has different genotypes for alleles. In addition, xenogenic mitochondria refer to mitochondria obtained from an individual that belongs to a different species from the individual. Furthermore, not only mitochondria in intact state may be used, but also crushed mitochondria may be included.

Specifically, the somatic cells may be muscle cells, hepatocytes, neurons, fibroblasts, epithelial cells, adipocytes, osteocytes, leukocytes, lymphocytes, or mucosal cells. In addition, the germ cells may be cells that undergo meiosis and mitosis, including sperms or eggs. In addition, the stem cells may be any one selected from the group consisting of mesenchymal stem cells, adult stem cells, induced pluripotent stem cells, embryonic stem cells, bone marrow stem cells, neural stem cells, limbal stem cells, and stem cells derived from tissues. Here, the mesenchymal stem cells may be derived from any one selected from the group consisting of umbilical cord, umbilical cord blood, bone marrow, fat, muscle, nerve, skin, amniotic membrane, and placenta.

Meanwhile, when the mitochondria are isolated from specific cells, the mitochondria may be isolated, through various known methods, for example, using a specific buffer solution or using a potential difference and a magnetic field.

The mitochondrial isolation may be achieved by crushing cells and performing centrifugation, in terms of maintaining mitochondrial activity. In an embodiment, mitochondrial isolation may be performed by a method that comprises a step of culturing cells and subjecting a pharmaceutical composition containing such cells to a first centrifugation to produce a pellet, a step of resuspending the pellet in a buffer solution and performing homogenization, a step of subjecting the homogenized solution to a second centrifugation to prepare supernatant, and a step of subjecting the supernatant to a third centrifugation to purify mitochondria. Here, it is preferable, in terms of maintaining cell activity, that the time during which the second centrifugation is performed is regulated to be shorter than the time during which the first and third centrifugations are performed. A centrifugation speed may be increased as centrifugation proceeds from the first centrifugation to the third centrifugation.

Specifically, the first to third centrifugations may be performed at a temperature of 0°C to 10°C, preferably at a temperature of 3°C to 8°C. In addition, the time during which the centrifugation is performed may be 1 to 50 minutes, and may be appropriately adjusted depending on the number of centrifugations, the amount of a sample, and the like.

In addition, the first centrifugation may be performed at a speed of 100×*g* to 1,000×*g*, or 200×*g* to 700×*g*, or 300×*g* to 450×*g*. In addition, the second centrifugation may be performed at a speed of 1×*g* to 2,000×*g*, or 25×*g* to 1,800×*g*, or 500×*g* to 1,600×*g*. In addition, the third centrifugation may be performed at a speed of 100×*g* to 20,000×*g*, or 500×*g* to 18,000×*g*, or 800×*g* to 15,000×*g*.

Mitochondria may be quantified by measuring the amount of membrane proteins of the isolated mitochondria. Specifically, the isolated mitochondria may be quantified through bicinchoninic acid assay (BCA). Here, mitochondria may be contained in a pharmaceutical composition at a concentration of 0.1 µg/mL to 1,000 µg/mL, 1 µg/mL to 750 µg/mL, or 25 µg/mL to 500 µg/mL. In an embodiment of the present invention, mitochondria were used at a concentration of 25 µg/mL, 50 µg/mL, or 100 µg/mL.

In addition, the number of isolated mitochondria may be measured through a particle counter (Multisizer 4e, Beckman Coulter). According to the James D. McCully's article *(*J Vis Exp. 2014; (91): 51682), the number of mitochondria may be as shown in Table 1 below.

**[Table 1]**

| Amount of isolated mitochondria (µg) | Number of mitochondria | Concentration (µg/mL) |
|---|---|---|
| 0.01 | 2.16 × 10⁵ ± 0.01 × 10⁶ | 0.1 |
| 1 | 2.16 × 10⁷ ± 0.08 × 10⁷ | 10 |
| 25 | 0.54 × 10⁹ ± 0.02 × 10⁹ | 250 |
| 50 | 1.08 × 10⁹ ± 0.04 × 10⁹ | 500 |
| 100 | 2.16 × 10⁹ ± 0.08 × 10⁹ | 1,000 |

As shown in Example 12 of the specification, the number of mitochondria for 1 µg/mL, 2.5 µg/mL, and 5 µg/mL was measured using a particle counter. As a result, the number of mitochondria was measured as 1.96×10⁶ ± 0.98×10⁶, 5.97×10⁶ ± 0.19×10⁶, and 1.01×10⁷ ± 0.32×10⁷, respectively. When referring to the values of Table 1 above, the number of mitochondria at a concentration of 10 µg/mL is 2.16×10⁷ ± 0.08×10⁷. This is found to have a range similar to 2.02×10⁷ ± 0.64×10⁷, which is obtained by multiplying the number of mitochondria at a concentration of 5 µg/mL by a factor of 2. Here, mitochondria may be contained in an amount of 1×10⁵ mitochondria/mL to 5×10⁹ mitochondria/mL in a pharmaceutical composition. Specifically, mitochondria may be contained, in a pharmaceutical composition, in an amount of 1×10⁵ mitochondria/mL to 5×10⁹ mitochondria/mL, 2×10⁵ mitochondria/mL to 2×10⁹ mitochondria/mL, 5×10⁵ mitochondria/ml to 1×10⁹ mitochondria/mL, 1×10⁶ mitochondria/mL to 5×10⁸ mitochondria/mL, 2×10⁶ mitochondria/mL to 2×10⁸ mitochondria/mL, 5×10⁶ mitochondria/mL to 1×10⁸ mitochondria/mL, or 1×10⁷ mitochondria/mL to 5×10⁷ mitochondria/mL.

When a pharmaceutical composition contains mitochondria at concentrations and amounts in the above ranges, a mitochondrial dose may be easily regulated in administration, and an amelioration degree of patient's sepsis or SIRS may be further improved.

In particular, a therapeutically effective dose of mitochondria to be contained in a pharmaceutical composition may be 3×10⁵ mitochondria/kg to 1.5×10¹⁰ mitochondria/kg per single administration, based on the body weight of an individual to which the pharmaceutical composition is to be administered. Specifically, a therapeutically effective dose of mitochondria in a pharmaceutical composition may be 3×10⁵ mitochondria/kg to 1.5×10¹⁰ mitochondria/kg, 6×10⁵ mitochondria/kg to 6×10⁹ mitochondria/kg, 1.5×10⁶ mitochondria/kg to 3×10⁹ mitochondria/kg, 3×10⁶ mitochondria/kg to 1.5×10⁹ mitochondria/kg, 6×10⁶ mitochondria/kg to 6×10⁸ mitochondria/kg, 1.5×10⁷ mitochondria/kg to 3×10⁸ mitochondria/kg, or 3×10⁷ mitochondria/kg to 1.5×10⁸ mitochondria/kg per single administration, based on the body weight of an individual to which the pharmaceutical composition is to be administered. In other words, it is most preferable, in terms of cell activity, that a pharmaceutical composition contains mitochondria such that the mitochondria are administered at doses in the above ranges based on the body weight of an individual having sepsis.

In addition, the pharmaceutical composition may be administered 1 to 10 times, 3 to 8 times, or 5 to 6 times, with 5 times being preferred. Here, administration may be performed at 1-to 7-day intervals or 2- to 5-day intervals, with 3-day intervals being preferred.

Furthermore, the pharmaceutical composition may also comprise one or more antibiotics. The antibiotics may be, but are not limited to, doripenem, cefepime, imipenem, meropenem, ceftazidime, ceftaroline fosamil, ceftriaxone, imipenem, vancomycin, teicoplanin, cefotaxime, metronidazole, penicillin or aminoglycoside-based antibiotics.

Furthermore, the pharmaceutical composition may also comprise one or more antiviral agents. The antiviral agents may be at least any one selected from the group consisting of oseltamivir, zanamivir, peramivir, acyclovir, valacyclovir, famciclovir, trifluridine, lamivudine, telbivudine, clevudine, entecavir, adefovir, tenofovir disoproxil, tenofovir alafenamide, besifovir, ribavirin, dasabuvir, sofosbuvir, daclatasvir, asunaprevir, zidovudine, abacavir, efavirenz, etravirine, nevirapine, rilpivirine, atazanavir, darunavir, nelfinavir, ritonavir, indinavir, dolutegravir, raltegravir, enfuvirtide, maraviroc and a combination thereof.

The pharmaceutical composition may contain one or more antibiotics or antiviral agents such that the antibiotics or the antiviral agents are administered in an amount of 0.1 mg/kg to 200 mg/kg per single administration, based on the body weight of an individual to which the pharmaceutical composition is to be administered. Specifically, the pharmaceutical composition may contain one or more antibiotics such that the antibiotics are administered in an amount of 0.1 mg/kg to 200 mg/kg, 1 mg/kg to 190 mg/kg, 5 mg/kg to 180 mg/kg, 10 mg/kg to 170 mg/kg, 20 mg/kg to 160 mg/kg, or 40 mg/kg to 150 mg/kg. In other words, it is most preferable, in terms of pharmacology, that the pharmaceutical composition contains one or more antibiotics such that the antibiotics are administered in the above amount ranges based on the body weight of an individual having sepsis. In an embodiment of the present invention, the antibiotics were administered in an amount of 150 mg/kg.

The pharmaceutical composition may be administered to humans or other mammals suffering from or suspected of having sepsis or SIRS. In addition, the pharmaceutical composition may be an injectable that can be administered intravenously, intramuscularly, or subcutaneously, and preferably may be an injectable preparation.

In order to ensure product stability during distribution of the injectables, the pharmaceutical composition may be prepared as an injectable which is physically or chemically very stable, by regulating pH, Redox, osmolality thereof using a buffer solution containing salts and sugars which can be used for injectables.

Specifically, the pharmaceutical composition of the present invention may contain water for injection. The water for injection means distilled water made to dissolve a solid injection or to dilute a water-soluble injection.

The pharmaceutical composition of the present invention may contain a stabilizer or a solubilizing agent. For example, the stabilizer may be pyrosulfite, citric acid or ethylenediaminetetraacetic acid, and the solubilizing agent may be hydrochloric acid, acetic acid, carbonic acid or potassium hydroxide.

### Kit for the treatment of sepsis or SIRS comprising mitochondria as an effective ingredient

In another aspect of the present invention, there is provided a kit for preventing or treating sepsis or SIRS, comprising a first composition containing mitochondria as an effective ingredient and a second composition containing one or more antibiotics or antiviral agents as an effective ingredient.

In this case, the mitochondria may be isolated mitochondria. The mitochondria, antibiotics and antiviral agents are as described above.

A therapeutically effective dose of mitochondria to be contained in the first composition may be 3×10⁵ mitochondria/kg to 1.5×10¹⁰ mitochondria/kg per single administration, based on the body weight of an individual to which the composition is to be administered. Specifically, a therapeutically effective dose of mitochondria in the composition may be 3×10⁵ mitochondria/kg to 1.5×10¹⁰ mitochondria/kg, 6×10⁵ mitochondria/kg to 6×10⁹ mitochondria/kg, 1.5×10⁶ mitochondria/kg to 3×10⁹ mitochondria/kg, 3×10⁶ mitochondria/kg to 1.5×10⁹ mitochondria/kg, 6×10⁶ mitochondria/kg to 6×10⁸ mitochondria/kg, 1.5×10⁷ mitochondria/kg to 3×10⁸ mitochondria/kg, or 3×10⁷ mitochondria/kg to 1.5×10⁸ mitochondria/kg per single administration, based on the body weight of an individual to which the composition is to be administered. That is, it is most preferable, in terms of cell activity, that the composition contains mitochondria such that the mitochondria are administered at doses in the above ranges based on the body weight of an individual having sepsis

In addition, the first composition may be administered 1 to 10 times, 3 to 8 times, or 5 to 6 times, with 5 times being preferred. Here, administration may be performed at 1- to 7-day intervals or 2- to 5-day intervals, with 3-day intervals being preferred.

The second composition may contain one or more antibiotics or antiviral agents such that the antibiotics or antiviral agents are administered in an amount of 0.1 mg/kg to 200 mg/kg per single administration, based on the body weight of an individual to which the composition is to be administered. Specifically, the second composition may contain one or more antibiotics or antiviral agents such that the antibiotics or antiviral agents are administered in an amount of 0.1 mg/kg to 200 mg/kg, 1 mg/kg to 190 mg/kg, 5 mg/kg to 180 mg/kg, 10 mg/kg to 170 mg/kg, 20 mg/kg to 160 mg/kg, or 40 mg/kg to 150 mg/kg. That is, it is most preferable, in terms of pharmacology, that the second composition contains one or more antibiotics or antiviral agents such that the antibiotics or antiviral agents are administered in amounts in the above ranges based on the body weight of an individual having sepsis. In an embodiment of the present invention, the antibiotics were administered in an amount of 150 mg/kg.

The first composition and the second composition may be administered simultaneously, sequentially, or in a reverse order. Specifically, the first and the second composition may be administered simultaneously. In addition, the first composition may be administered first, and followed by the second composition. Further, the second composition may be administered first, and followed by the first composition. Furthermore, the second composition may be administered first, and followed by the administration of the first composition and again the second composition.

The first composition and the second composition may be administered to humans or other mammals suffering from or suspected of having sepsis or SIRS. In addition, both compositions may be injectables that can be administered intravenously, intramuscularly, or subcutaneously, and preferably may be injectable preparations.

In order to ensure product stability during distribution of the injectables, the first and the second composition may be prepared as injectables which are physically or chemically very stable, by regulating pH, Redox, osmolality thereof using a buffer solution containing salts and sugars which can be used for injectables.

As used herein, the term "injection" or "injectable" refers to a sterile preparation of a pharmaceutical product applied directly into the body through the skin, or through the skin and mucous membranes in the form of a solution, suspension, emulsion, or those being dissolved or suspended upon use. Specifically, it may be used as an injection, powder injection, infusion, lyophilized injection, implant, persistent injection, peritoneal dialysis agent, perfusion or dialysis agent. Specifically, the injection may be administered as bolus, infusion, subcutaneous injection, or intramuscular injection.

Specifically, the first composition and the second composition may contain water for injection. The water for injection means distilled water made to dissolve a solid injection or to dilute a water-soluble injection.

The first and the second composition may contain a stabilizer or a solubilizing agent. For example, the stabilizer may be sodium pyrosulfite, citric acid or ethylenediaminetetraacetic acid, and the solubilizing agent may be hydrochloric acid, acetic acid, carbonic acid or potassium hydroxide.

### A method of treating sepsis or SIRS comprising a step of administering mitochondria

Another aspect of the present invention is to provide a method for treating sepsis or SIRS, comprising a step of administering to a subject a pharmaceutical composition as described above. Here, the subject may be a mammal, preferably a human.

In this case, antibiotics or antiviral agents may be administered simultaneously or sequentially. The dose of mitochondria used, the dose of antibiotic or antiviral agent is as described above.

Here, the administration may be intravenous, intramuscular, or intradermal administration. This allows a pharmaceutical composition according to the present invention to supply exogenous mitochondria having normal activity through the vein of a subject suffering from sepsis or SIRS. Therefore, the pharmaceutical composition may be useful to increase activity of cells with decreased mitochondrial function or to regenerate cells with mitochondrial dysfunction, and thus may be used to prevent or treat sepsis or SIRS.

### Use of mitochondria for the treatment of sepsis or SIRS

Another aspect of the present invention provides the use of mitochondria for treating sepsis or systemic inflammatory response syndrome (SIRS). Mitochondria, sepsis and SIRS are as described above.

In this case, antibiotics or antiviral agents may be administered simultaneously or sequentially for the purpose. The dose of mitochondria used, the dose of antibiotic or antiviral agent is as described above.

### Mode for Carrying Out the Invention

Hereinafter, preferred examples of the present invention will be described in order to facilitate understanding of the present invention. However, the following examples are provided only for the purpose of smooth understanding of the present invention which is not limited by the following examples

### I. Preparation of Composition Containing Mitochondria

### Preparation Example 1. Preparation of Composition Containing Mitochondria Isolated from Myoblast Cells Derived from Rat Skeletal Muscle

L6 cells (American Type Culture Collection, ATCC, CRL-1458), a myoblast cell line derived from rat skeletal muscle, were inoculated in Dulbecco's modified eagle's medium-High glucose (DMEM-High glucose, Gibco) supplemented with 10% fetal bovine serum (FBS, Gibco) and cultured for 72 hours. After the culture was completed, washing was performed twice using Dulbecco's phosphate buffered saline (DPBS, Gibco). Then, treatment with 0.25% trypsin-EDTA (TE) was performed to obtain cells. In order to extract mitochondria, the obtained cells were resuspended so that the cell concentration became 1×10⁷ cells/mL using hemocytometer.

Subsequently, the cells were subjected to a first centrifugation at a speed of 350×g for 10 minutes at a temperature of 4°C. Here, the obtained pellet was collected, resuspended in a buffer solution, and homogenized. A composition containing the pellet was subjected to a second centrifugation at a speed of 1,500×g for 5 minutes at a temperature of 4°C to obtain a supernatant. Thereafter, the supernatant was subjected to the third centrifugation at a speed of 20,000×g for 10 minutes at 4°C, to isolate mitochondria from the cell line. The isolated mitochondria were quantified in an amount such as 5 µg, 10 µg, 25 µg, 50 µg, or 100 µg using bicinchoninic acid assay (BCA), and the respective amounts of mitochondria were mixed with a solvent and used for each experiment.

### Preparation Example 2. Preparation of Composition Containing Mitochondria Isolated from Mesenchymal Stem Cells Derived from Human Umbilical Cord

Mesenchymal stem cells derived from human umbilical cord were inoculated in Alpha-Minimum essential medium (Alpha-MEM, Gibco) supplemented with 10% fetal bovine serum (FBS, Gibco), 100 µg/mL streptomycin, and 100 U/mL ampicillin, and cultured for 72 hours. After the culture of the cells was completed, washing was performed twice using DPBS. Thereafter, treatment with 0.25% trypsin-EDTA was performed to obtain cells. The cells were resuspended so that the cell concentration became 1×10⁷ cells/mL and a first centrifugation was performed at 4 °C for 10 minutes at a speed of 350g.

The washed cells were resuspended using a mitochondria isolation solution and then crushed using a 1 mL syringe. Thereafter, the solution containing the crushed cells was centrifuged at 4 °C for 5 minutes at 1,500 x g to remove impurities, and the supernatant containing mitochondria was recovered. The supernatant was centrifuged at 4 ° C for 5 minutes at 20,000xg to recover the precipitated mitochondria, and the isolated mitochondria were suspended in a Tris Buffer and used for experiments after quantifying the protein by BCA method.

### Preparation Example 3. Preparation of Composition Containing Mitochondria Isolated from Mesenchymal Stem Cells Derived from Human Bone Marrow

Mesenchymal stem cells derived from human bone marrow (BM-MSC) were inoculated in DMEM (Gibco) medium containing 10% fetal bovine serum (FBS, Gibco), 100 µg/mL streptomycin and 100 U/mL ampicillin and cultured for 72 hours.

After the culture of the cells was completed, the mitochondria were recovered and quantified as in the method described in Preparation Example 2, and then used in the experiment.

### Preparation Example 4. Preparation of Composition Containing Mitochondria Isolated from Human Fibroblasts

Human fibroblasts (CCD-8LU, ATCC) were inoculated in DMEM (Gibco) medium containing 10% fetal bovine serum (FBS, Gibco), 100 µg/mL streptomycin and 100 U/mL ampicillin and cultured for 72 hours.

After the culture of the cells was completed, the mitochondria were recovered and quantified as in the method described in Preparation Example 2, and then used in the experiment.

### Preparation Example 5. Preparation of Composition Containing Mitochondria Isolated from Human Induced Pluripotent Stem Cell (iPS)

Human induced pluripotent stem cells (iPSC) were cultured in TeSRTM-E8TM (stem cell 05990) medium in a cell culture container coated with 10 ug/mL of vitronectin (stem cell 07180).

After the culture of the cells was completed, the mitochondria were recovered and quantified as in the method described in Preparation Example 2, and then used in the experiment.

### Preparation Example 6. Preparation of Composition Containing Mitochondria Isolated from Platelets

To isolate mitochondria from platelets, the porcine whole blood was centrifuged at 500xg for 3 minutes at room temperature, and then the supernatant containing platelet-rich plasma (PRP) was recovered. The recovered supernatant was centrifuged at 1,500xg for 5 minutes to remove the supernatant, and the precipitate containing platelets was recovered. The concentrated platelet precipitate was resuspended using PBS and washed by centrifugation at 1,500xg for 5 minutes. The washed platelets were resuspended using a mitochondria isolation solution, and then crushed using a 1 mL syringe. Afterwards, the solution containing the crushed platelets was centrifuged at 4°C for 5 minutes at 1,500xg to remove impurities, and the supernatant containing mitochondria was recovered. The recovered supernatant was collected by centrifugation at 4°C for 5 minutes at 20,000xg to recover the precipitated mitochondria. The isolated mitochondria were suspended in a Tris Buffer and used for the experiment after quantifying the protein.

### Preparation Example 7. Preparation of Composition Containing Mitochondria Isolated from Myoblasts Derived from Rat Skeletal Muscle

L6 cells (American Type Culture Collection, ATCC, CRL-1458), a myoblast cell line derived from rat skeletal muscle, were inoculated in DMEM-high glucose (Dulbecco's modified eagle's medium-High glucose, Gibco) medium containing 10% fetal bovine serum (FBS, Gibco) and cultured for 72 hours.

After the culture of the cells was completed, the mitochondria were recovered and quantified as in the method described in Preparation Example 2, and then used in the experiment.

### II. In Vitro Confirmation of Anti-Inflammatory Effect by Mitochondria

### Example 1. Inhibitory Effect on Pro-Inflammatory Cytokine Secretion of Macrophages by Delivery of Mitochondria

By the same method as Example 1, mouse Raw 264.7 cells were subjected to treatment with 2 µg/mL of LPS, and inflammatory responses were induced for 6 hours. Then, the cells were treated with mitochondria of 0.1 µg, 0.5 µg, 0.75 µg, 1 µg, or 5 µg extracted in Preparation Example 2, and then inoculated in a 24-well-plate. After 24 hours, the supernatant was obtained. The supernatant was subjected to centrifugation at 1,500 rpm for 5 minutes, and only the supernatant was transferred to a new tube.

In order to confirm the expression levels of TNF-α and IL-6, which are pro-inflammatory cytokines of macrophage cells, analysis was performed using TNF-α ELISA kit (Sigma, #RAB0477) and IL-6 ELISA kit (Sigma, #RAB0308) respectively. A supernatant sample for each condition was prepared by being diluted at a ratio of 1/10 using a dilution solution included in the ELISA assay kit. The prepared sample and a standard solution were respectively placed in a 96-well-plate pre-coated with antibodies, and reaction was allowed to proceed overnight at 4°C.

Thereafter, the 96-well-plate was washed using a washing solution included in the assay kit. After the washing, each well was treated with biotin-labeled antibodies (detection antibodies), and then reaction was allowed to proceed at room temperature for 1 hour. Subsequently, washing was performed using a washing solution, and then a streptavidin solution was injected into each well. Then, reaction was allowed to proceed at room temperature for 45 minutes. Subsequently, washing was performed using a washing solution, and then a substrate solution was added. Reaction was allowed to proceed for 30 minutes in a light shielding environment at room temperature. Then, a stop solution was added to each well, and absorbance was measured at a wavelength of 450 nm.

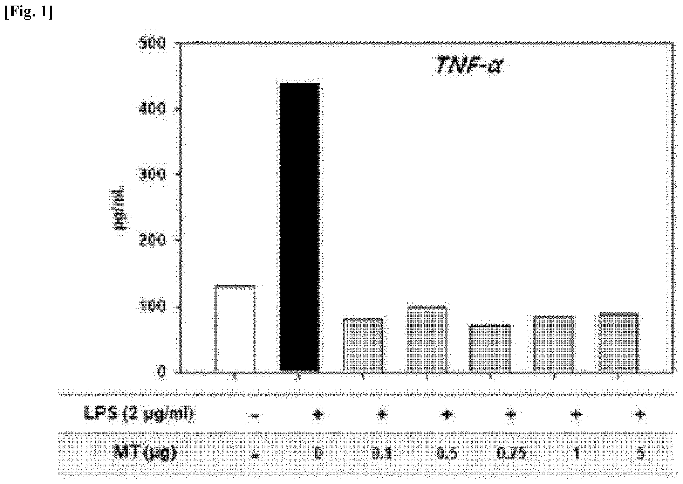

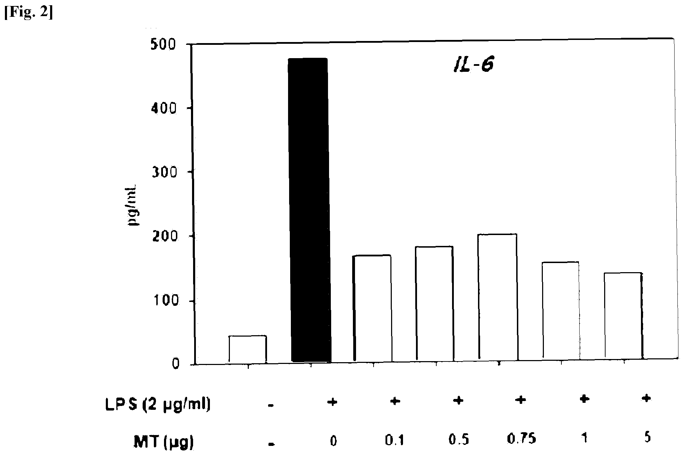

As a result, in case of the Raw 264.7 cells treated with LPS only, secretion levels of TNF-α and IL-6 were increased. On the other hand, in case of the Raw 264.7 cells treated with mitochondria, secretion levels of TNF-α and IL-6 were remarkably decreased to the levels below normal cells in spite of the same LPS treatment (Figs. 1 and 2).

### Example 2. Comparison of Anti-Inflammatory Activity in Human Monocytes (THP-1) by Mitochondria Derived from Various Types of Cells

Human monocytes THP-1 cells were cultured in RPMI medium containing 10% FBS. Cells in an amount of 4×10⁵ cells / well were inoculated into a 24 well plate and cultured for 15 to 16 hours in RPMI medium containing 1% FBS.

Inflammatory response in the THP-1 cell line was induced by treatment with Salmonella-derived lipopolysaccharide (LPS) at a concentration of 2 µg / mL for 6 hours. After 6 hours of lipopolysaccharide treatment, the THP-1 cells were treated with mitochondria obtained from each cell of Preparation Example 2, Preparation Example 4, Preparation Example 5 and Preparation Example 6 and further cultured for 24 hours. The negative control group is a group that is not treated with lipopolysaccharide and mitochondria, and the positive control group is a group that is treated with lipopolysaccharide at a concentration of 2 µg/mL alone. In addition, the experimental group was treated with lipopolysaccharide at a concentration of 2 µg/mL, and 6 hours later, treated with 40 µg of mitochondria obtained each from mesenchymal stem cells derived from umbilical cord (UC-MSC), fibroblasts derived from human lung (CCD-8LU), human induced pluripotent stem cells (IPS), and porcine platelets (Platelet). To compare the anti-inflammatory activity after the reaction, the cells were used for a quantitative real-time polymerization chain reaction method, and the culture medium was used for an ELISA method.

### Example 2.1. Comparison of Anti-Inflammatory Activity Using Quantitative Real-Time Polymerization Chain Reaction

Thereafter, the culture solution was removed, and PBS buffer solution was added to wash the cells twice, and 0.5 mL of RNA extract (Trizol reagent, Thermo Fisher Scientific) was added directly. After standing at room temperature for 10 minutes, 0.1 mL of chloroform was added and stirred for 15 seconds, followed by centrifugation at 12,000xg for 10 minutes. The separated supernatant was taken and the same volume of isopropyl alcohol was added and centrifuged at 12,000xg for 10 minutes. After that, the supernatant was removed, washed once with 75% ethanol, and dried at room temperature.

50 µL of RNAse-free purified distilled water was added, and the quantity and purity of the obtained RNA was measured using a spectrophotometer. To synthesize cDNA, 2 µg of purified total RNA was subjected to a binding reaction with oligo dT at 70 °C. for 5 minutes. Afterward, cDNA synthesis reaction was performed at 42 °C for 60 minutes by adding 10X reverse transcription buffer solution, 10 mM dNTP, RNAse inhibitor, and M-MLV reverse transcriptase (Enzynomics, Korea). After the reaction, heating was performed at 72 °C for 5 minutes to inactivate the reverse transcriptase, and then RNase H was added to remove single-stranded RNA to obtain cDNA.

Quantitative RT-PCR was performed using the primers shown in Table 2 below to determine whether cytokine expression of pre-inflammatory factors was changed. The difference in gene expression was corrected by quantifying with 18S.

**[Table 2]**

| **Primer** | **Nucleic Sequence** |
|---|---|
| **Human IL-6-S** | ccacacagacagccactcac (SEQ ID NO: 1) |
| **Human IL-6-AS** | tttcaccaggcaagtctcct (SEQ ID NO: 2) |
| **Human 18S-S** | ctcccacttggataactgtgg (SEQ ID NO: 3) |
| **Human 18S-AS** | gaccgggttggttttgatct (SEQ ID NO: 4) |

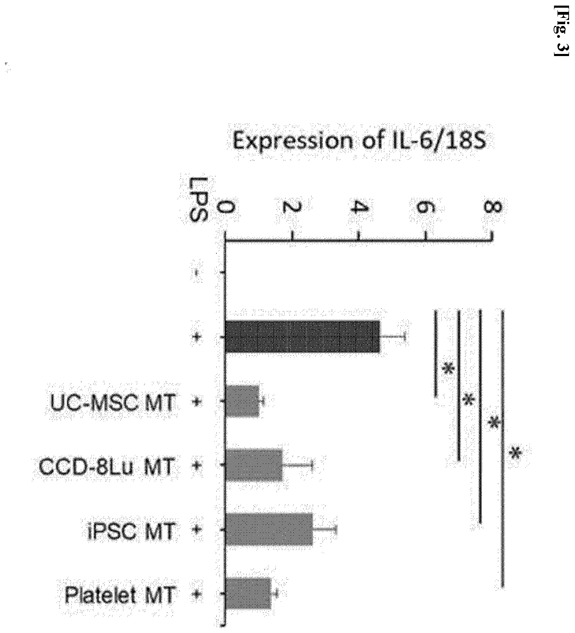

As shown in the results of Fig. 3, it was found that expression of the IL-6 gene increased in THP-1 cells, which are human monocytes, treated with lipopolysaccharide. In addition, it was confirmed that the expression of IL-6 gene induced by lipopolysaccharide was suppressed to a significant level when treated with mitochondria obtained from mesenchymal stem cells derived from umbilical cord, fibroblasts derived from human lung, human induced pluripotent stem cells, and porcine platelets. Through this, it was confirmed that the mitochondria obtained from various cells exhibit a remarkably excellent anti-inflammatory activity (Fig. 3, ^{∗} P <0.05).

### Example 2.2. Comparison of Anti-Inflammatory Activity Using ELISA Method

In order to confirm the expression level of IL-6, a pro-inflammatory cytokine of THP-1 cells in the obtained supernatant, the experiment was performed using human IL-6 (R&D Systems) according to the manufacturer's manual as follows.

The 100 µL coating solution was put into a 96-well-plate, reacted overnight at room temperature, then washed 3 times, then reacted with a reagent diluent for 1 hour at room temperature, and washed 3 times. The 10-fold diluted supernatant and the standard solution were reacted at room temperature for 2 hours, washed 3 times, and then treated with the labeled antibody (detection antibody) in each well, and then reacted at room temperature for 2 hours. After washing 3 times, the streptavidin solution (streptavidin-HRP) was reacted at room temperature for 20 minutes, then washed three times, then reacted with the color solution (substrate solution) for 20 minutes in a dark room at a room temperature, and then the reaction stop solution was added. Absorbance was measured at the wavelength of 450 nm.

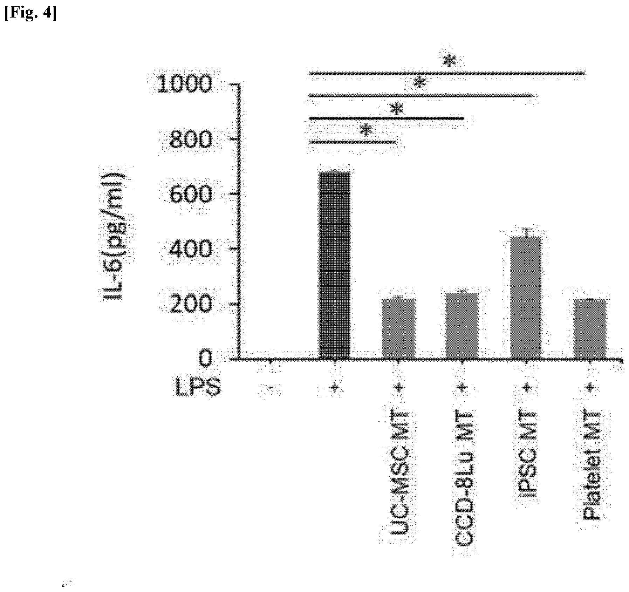

As shown in the results of Fig. 4, it was found that IL-6 protein increased when THP-1 cells were treated with lipopolysaccharides. In addition, it was confirmed that IL-6 protein induced by lipopolysaccharide was inhibited to a significant level when treated mitochondria obtained from mesenchymal stem cells derived from umbilical cord, fibroblasts derived from human lung, human induced pluripotent stem cells, and porcine platelets. This was consistent with the results of gene expression. Through this, it was confirmed that the mitochondria obtained from various cells showed a remarkably excellent anti-inflammatory activity (Fig. 4, ^{∗} P <0.05).

### Example 3. Comparison of Anti-Inflammatory Activity in RAW264.7 Cells by Mitochondria Derived from Various Types of Cells Using Quantitative Real-Time Polymerization Chain Reaction : In Vitro

A cell-based analysis experiment using a quantitative real-time polymerization chain reaction method was performed in order to compare and analyze the anti-inflammatory activity by mitochondria obtained from various cells of Preparation Example 2, Preparation Example 3, Preparation Example 4 and Preparation Example 7.

RAW264.7 cells, a mouse macrophage cell line, were cultured in DMEM medium containing 10% FBS. Around 3×10⁵ cells / well were inoculated into a 6 well plate and cultured for 24 hours, then subjected to deficiency conditions in DMEM medium from which FBS was removed for about 24 hours.

After 24 hours, the macrophage cell line was treated with salmonella-derived lipopolysaccharide (LPS) at a concentration of 1 µg/mL for 6 hours to induce an inflammatory reaction. After 6 hours of lipopolysaccharide treatment, the cells were washed twice with PBS buffer solution, and then treated with the mitochondria obtained from each cell and further cultured for 18 hours. At this time, the negative control group is a group that is not treated with lipopolysaccharide and mitochondria, and the positive control group is a group that is treated with lipopolysaccharide at a concentration of 1 µg/mL alone. In addition, the experimental group was treated with lipopolysaccharide at a concentration of 1 µg/mL, and 6 hours later, treated with 30 µg of mitochondria obtained each from mesenchymal stem cells derived from bone marrow (BM-MSC), mesenchymal stem cells derived from umbilical cord (UC-MSC), myoblasts derived from rat skeletal muscle (L6 myoblast), and fibroblasts derived from human lung (CCD-8LU).

After 18 hours of treatment with mitochondria, the culture solution was removed, and the cells were washed twice by adding PBS buffer solution. 0.5 mL of RNA extract (Trizol reagent, Thermo Fisher Scientific) was added directly, and then allowed to stand at room temperature for 10 minutes. Thereafter, 0.1 mL of chloroform was added and stirred for 15 seconds, and then centrifuged at about 12,000xg for 10 minutes.

The separated supernatant was obtained, and the same volume of isopropyl alcohol was added, followed by centrifugation at 12,000xg for 10 minutes. Then, the liquid was removed and washed once with 75% ethanol, and then dried at room temperature. After drying, about 50 µL of RNAse-free purified distilled water was added, and the quantity and purity of the obtained RNA was measured using a spectrophotometer.

To synthesize cDNA using the obtained RNA, 2 µg of purified total RNA was subjected to a binding reaction with oligo dT at 70 °C. for 5 minutes. Afterward, cDNA synthesis reaction was performed at 42 °C for 60 minutes by adding 10X reverse transcription buffer solution, 10 mM dNTP, RNAse inhibitor, and M-MLV reverse transcriptase (Enzynomics, Korea).

After the cDNA synthesis reaction was completed, the transcriptase was inactivated by heating at 72 °C for 5 minutes, and then RNase H was added to remove single-stranded RNA to obtain a final cDNA. Changes in the expression of TNF-α, IL-Iβ and IL-6 genes, which are characteristic genes of the inflammatory response, were observed through quantitative real-time polymerization chain reaction. GAPDH gene was quantified together to correct the difference in expression. The nucleic sequences of the genes used in the quantitative real-time polymerization chain reaction are shown in Table 3 below.

**[Table 3]**

| **Primer** | **Sequence** |
|---|---|
| TNF-alpha-S | 5'-TCTCATCAGTTCTATGGCCC-3' (SEQ ID NO: 5) |
| TNF-alpha-AS | 5'-GGGAGTAGACAAGGTACAAC-3' (SEQ ID NO: 6) |
| IL-1 beta-F | 5'-AACCTGCTGGTGTGTGACGTTC-3' (SEQ ID NO: 7) |
| IL-1 beta-R | 5'-CAGCACGAGGCTTTTTTGTTGT-3' (SEQ ID NO: 8) |
| IL-6-AS | 5'-CTAGGTTTGCCGAGTAGATCT-3' (SEQ ID NO: 9) |
| IL-6-S | 5'-CCAAACTGGATATAATCAGGAAAT -3' (SEQ ID NO: 10) |
| GAPDH-S | 5'-GGTGAAGGTCGGTGTGAACG-3' (SEQ ID NO: 11) |
| GAPDH-AS | 5'-CTCGCTCCTGGAAGATGGTG-3' (SEQ ID NO: 12) |

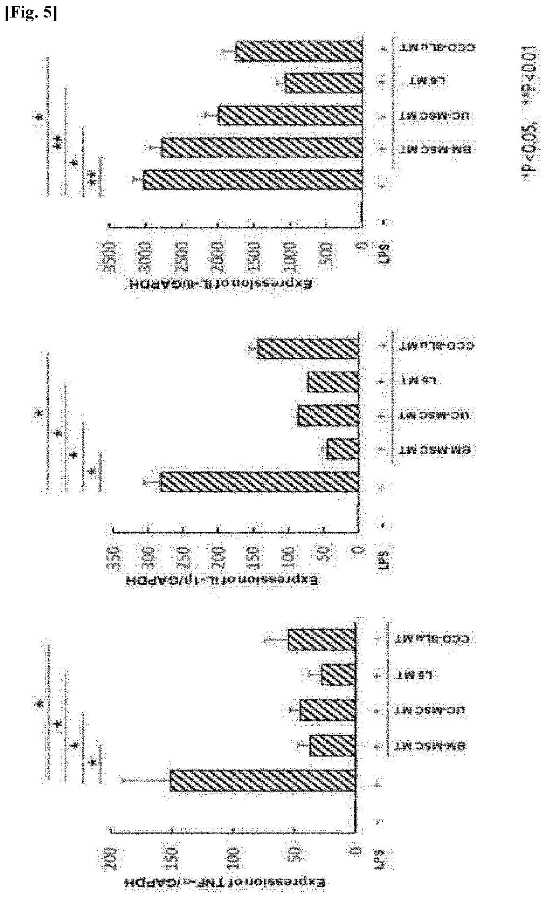

As shown in the experimental results, it was found that the expression of TNF-α, IL-Iβ and IL-6 genes increased when RAW 264.7 cells, a mouse macrophage cell line, were treated with lipopolysaccharide. In addition, it was confirmed that the expression of the TNF-α, IL-Iβ and IL-6 genes induced by lipopolysaccharide was suppressed to a significant level when treated with mitochondria obtained from mesenchymal stem cells derived from bone marrow (BM-MSC), mesenchymal stem cells derived from umbilical cord (UC-MSC), myoblasts derived from rat skeletal muscle (L6) and fibroblasts derived from human lung (CCD-8LU). Through this, it was confirmed that the mitochondria obtained from the cells used in the present invention exhibit remarkably excellent anti-inflammatory activity (Fig. 5).

### III. In Vivo Confirmation of Anti-Inflammatory Effect by Mitochondria in Animal Experiments

### Example 4. Efficacy Test for Single Intravenous Administration of Mitochondria Isolated from Mesenchymal Stem Cells Derived from Human Umbilical Cord (UC-MSC) in LPS-Induced Sepsis Mouse Model

The effect of improvement of the survival rate by mitochondria prepared in Example 2 was confirmed using the LPS-induced mouse sepsis model. A 9-week-old mouse (male Balb / c) was used as the experimental animal, and after measuring the body weights of mice, mice were assigned to each group so that the average value between groups was uniform. 100 µL of 7.5 mg/kg LPS (Lipopolysaccharides from Escherichia coli 011: B4) was administered intraperitoneally to each individual in the control group (n = 10) and the experimental group (n = 7).

6 hours after LPS administration, the control group was injected with 100 µL of buffer solution into the tail vein, and the experimental group was injected with 100 µL (10 µL/mouse) of mitochondria isolated from mesenchymal stem cells derived from umbilical cord (UC-MSC) into the tail vein. Survival rate was measured at a predetermined time interval after LPS administration, and efficacy of mitochondria administration was confirmed by comparing the survival rate.

As a result of measuring the survival rate, as shown in the table, it was observed that all subjects died at 33 hours in the control group after LPS administration. Compared to the control group, the test group injected with mitochondria showed a high survival rate of 71% at 33 hours, and it was confirmed that the survival rate of 57% was maintained from 33 hours to 72 hours when observation was terminated.

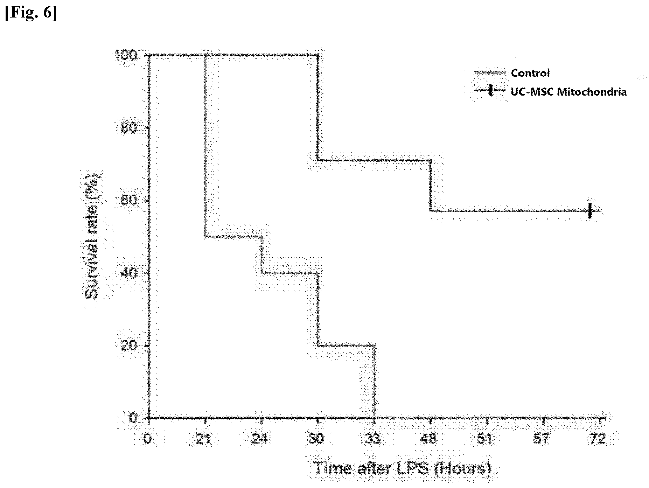

Through this, it was confirmed that mitochondria obtained from mesenchymal stem cells derived from human umbilical cord (UC-MSC) used in the present invention exhibited excellent efficacy by single intravenous administration in the sepsis mouse model. Table 4 below shows the survival rate by mitochondria derived from UC-MSC in the LPS-induced sepsis mouse model, and the results are summarized in Fig. 6 (Fig. 6).

**[Table 4]**

| Category | Survival rate % (No. of mice surviving /No. of total mice) | | | | | | | |
|---|---|---|---|---|---|---|---|---|
| | 21 hr | 24 hr | 30 hr | 33 hr | 48 hr | 51 hr | 57 hr | 72 hr |
| Control group(n=10) | 50 (5/10) | 40 (4/10) | 20 (2/10) | 0 (0/10) | 0 (0/10) | 0 (0/10) | 0 (0/10) | 0 (0/10) |
| Experimental group (n=7, UC-MSC mitochondria) | 100 (7/7) | 100 (7/7) | 71 (5/7) | 71 (5/7) | 57 (4/7) | 57 (4/7) | 57 (4/7) | 57 (4/7) |

### Example 5. Efficacy Test for Single Intravenous Administration of Mitochondria Derived from Platelets in LPS-Induced Sepsis Mouse Model

The effect of improvement of the survival rate by mitochondria prepared in Preparation Example 6 was confirmed using the LPS-induced mouse sepsis model. A 9-week-old mouse (male Balb / c) was used as the experimental animal, and after measuring the body weights of mice, mice were assigned to each group so that the average value between groups was uniform. 100 µL of 7.5 mg / kg LPS (Lipopolysaccharides from Escherichia coli 011: B4) was administered intraperitoneally to each individual in the control group (n = 10) and the experimental group (n = 7).

6 hours after LPS administration, the control group was injected with 100 µL of buffer solution into the tail vein, and the experimental group was injected with 100 µL (10 µL/mouse) of mitochondria isolated from platelets into the tail vein. Survival rate was measured at a predetermined time interval up to 72 hours after LPS administration and efficacy of mitochondria administration was confirmed by comparing the survival rate.

Through this, it was confirmed that mitochondria obtained from platelets used in the present invention also showed efficacy as in Example 4 in the LPS-induced sepsis mouse model.

### Example 6. Comparison I of Therapeutic Effects on Sepsis in Accordance with Administration of Mitochondria Alone and Combination of Mitochondria/Antibiotic

In order to confirm therapeutic effects on sepsis by administration of mitochondria isolated in Preparation Example 1 alone and combination of the mitochondria and an antibiotic, SD rats weighing 300 g to 350 g were anesthetized with gas inhalation, and then cecal slurry, obtained by diluting stool squeezed out of cecum of an allogenic animal in 5% dextrose solution at a ratio of 1:3, was intraperitoneally administered at a dose of 6 mL/kg to experimental animals, to induce polymicrobial sepsis. Immediately after sepsis induction, physiological saline was subcutaneously injected at a dose of 30 mL/kg weight of experimental animal, to replenish body fluid.

A group having received 0.5 mL of physiological saline only at 1 hour and 7 hours respectively after sepsis induction was designated as a complete placebo group; and a group having received, by intravenous injection via the tail vein, 50 µg of mitochondria suspended in 0.5 mL physiological saline at 1 hour and 7 hours respectively after sepsis induction was designated as an administration group of mitochondria alone. In addition, a group having received, by intramuscular injection, the antibiotics ceftriaxone at a dose of 150 mg/kg at 1 hour and 7 hours respectively after sepsis induction was designated as an administration group of an antibiotic alone.

The group having received, by intravenous injection, 50 µg of mitochondria suspended in 0.5 mL physiological saline and having received, by intramuscular injection, ceftriaxone at a dose of 150 mg/kg respectively after sepsis induction was designated as an administration group of the combination. For the rats in each group (n = 4), survival was observed for 14 days after the sepsis induction date, and then the observation was terminated. A mean survival time was analyzed as of the termination time point.

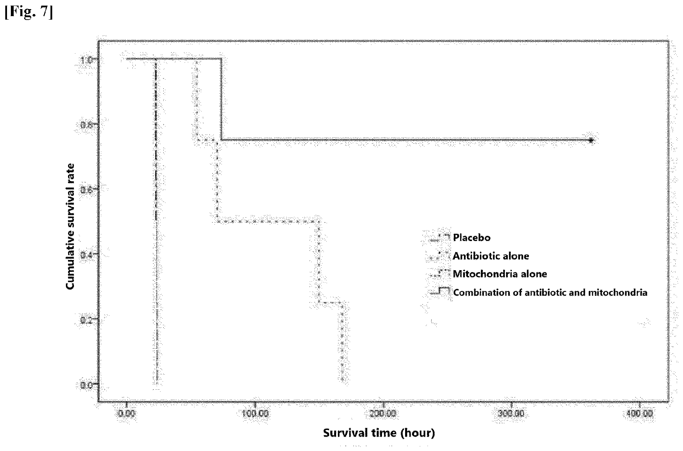

As a result, the mean survival time of the complete placebo group was 22.95 hours; the mean survival time of the administration group of mitochondrial alone was 22.99 hours; the mean survival time of the administration group of an antibiotic alone was 110.67 hours; and the mean survival time of the administration group of the combination of mitochondria and an antibiotic was 290.10 hours. In particular, it was found that when compared with the administration group of an antibiotic alone, the administration group of the combination exhibited a more than 2-fold increase in survival time (Fig. 7).

### Example 7. Comparison II of Therapeutic Effects on Sepsis in Accordance with Administration of Combination of Mitochondria/Antibiotic

In order to confirm therapeutic effects on sepsis in accordance with administration of the mitochondria isolated in Preparation Example 1, SD rats weighing 300 g to 350 g were anesthetized with gas inhalation, and then cecal slurry, obtained by diluting stool squeezed out of cecum of an allogenic animal in 5% dextrose solution at a ratio of 1:3, was intraperitoneally administered at a dose of 6 mL/kg to the experimental animals, to induce polymicrobial sepsis. Immediately after sepsis induction, the antibiotic ceftriaxone was intramuscularly injected at a dose of 150 mg/kg, and physiological saline was subcutaneously injected at a dose of 30 mL/kg weight of experimental animal, to replenish body fluid.

A group having received only 0.5 mL of physiological saline only at 1 hour and 7 hours respectively after sepsis induction was designated as a treatment group of placebo and an antibiotic (n = 18). In addition, a group having received, by intravenous injection, 50 µg of mitochondria suspended in 0.5 mL physiological saline at 1 hour and 7 hours respectively after sepsis induction was designated as an administration group of the combination of mitochondria and an antibiotic (n = 17). For the experimental animals in each group, survival was observed for 14 days after the sepsis induction date, and then the observation was terminated. A median value of estimated mean survival time was analyzed, through Kaplan-Meier survival analysis, as of the termination time point.

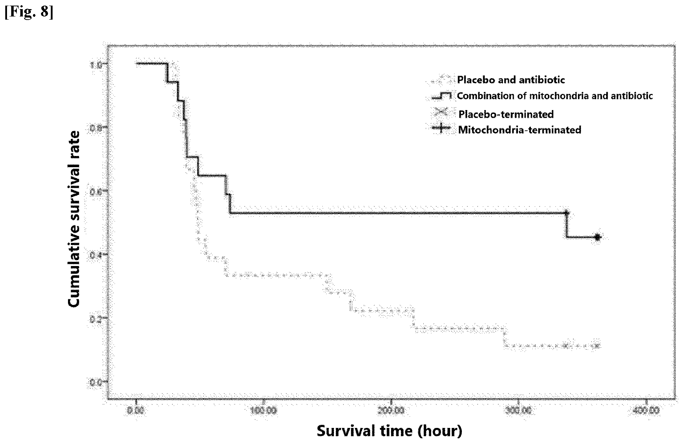

As a result, the median value of estimated survival time of the administration group of the combination of mitochondria and an antibiotic was analyzed as 337.37 hours, and the median value of estimated survival time of the treatment group of placebo and an antibiotic was analyzed as 48.23 hours. In a log rank test for the administration group of the combination of mitochondria and an antibiotic and the treatment group of placebo and an antibiotic, the p value was 0.032, indicating that there was a significant difference in survival time between the two groups (Fig. 8).

### Example 8. Comparison III of Therapeutic Effects in Sepsis Animal Model in Accordance with Administration of Combination of Mitochondria/Antibiotic

In order to confirm therapeutic effects on sepsis in accordance with administration of the mitochondria isolated in Preparation Example 2, SD rats weighing 300 g to 350 g were anesthetized with gas inhalation, and then cecal slurry, obtained by diluting stool squeezed out of cecum of an allogenic animal in 5% dextrose solution at a ratio of 1:3, was intraperitoneally administered at a dose of 6 mL/kg to the experimental animals, to induce polymicrobial sepsis. Immediately after sepsis induction, physiological saline was subcutaneously injected at a dose of 30 mL/kg weight of experimental animal, to replenish body fluid.

A group having received, by intramuscular injection, the antibiotic prepenem (imipenem series) at a dose of 25 mg/kg at the time of 1 hour and 6 hours after sepsis induction was designated as an administration group of an antibiotic alone. In addition, a group having received, by intravenous injection, 50 µg of mitochondria suspended in 0.5 mL physiological saline and having received, by intramuscular injection, 25 mg/kg of prepenem respectively at the time of 1 hour and 6 hours after sepsis induction was designated as an administration group of the combination.

Survival of each group (n = 6) of rats was observed for 14 days after the induction of sepsis date and then the observation was terminated. A mean survival time was analyzed as of the termination time point. As a result, as shown in Examples 5 and 6, the administration group of the combination of mitochondria and an antibiotic was confirmed to have an increased survival time compared to the administration group of an antibiotic alone.

### Example 9. Comparison IV of Therapeutic Effects in Sepsis Animal Model in Accordance with Administration of Combination of Mitochondria/Antibiotic

In order to confirm therapeutic effects on sepsis in accordance with administration of mitochondria isolated in Preparation Example 2, SD rats weighing 300 g to 350 g were anesthetized with gas inhalation, and then cecal slurry, obtained by diluting stool squeezed out of cecum of an allogenic animal in 5% dextrose solution at a ratio of 1:3, was intraperitoneally administered at a dose of 6 mL/kg to the experimental animals, to induce polymicrobial sepsis. Immediately after sepsis induction, physiological saline was subcutaneously injected at a dose of 30 mL/kg weight of experimental animal, to replenish body fluid.

A group having received, by intramuscular injection, the antibiotic tabaxin at a dose of 25 mg/kg at the time of 1 hour and 6 hours after sepsis induction was designated as an administration group of an antibiotic alone. In addition, a group having received, by intravenous injection, 50 µg of mitochondria suspended in 0.5 mL physiological saline and having received, by intramuscular injection, 25 mg/kg of tabaxin respectively at the time of 1 hour and 6 hours after sepsis induction was designated as an administration group of the combination.

Survival of each group (n = 6) of rats was observed for 14 days after the induction of sepsis date and then the observation was terminated. A mean survival time was analyzed as of the termination time point

As a result, as shown in Examples 5 and 6, the administration group of the combination of mitochondria and an antibiotic was confirmed to have an increased survival time compared to the administration group of an antibiotic alone.

### IV. Confirmation of Toxicity and Physical Properties of Compositions Containing Mitochondria

### Example 10. Toxicity Test

In order to identify whether toxicity is observed upon administration of mitochondria, mitochondria were intravenously administered to ICR mice once, and then changes in body weight, changes in organs through autopsy and the like were checked. Twelve 7-week-old ICR mice (male and female each) were divided into four groups as shown in Table 5 below, and experiments were carried out.

**[Table 5]**

| **Group** | **Sex** | **No. of individuals** | **Substance administered** | **Route of administration** | **Dose (µg/individual)** | **Dose (mL/individual)** | **Concentration (µg/mL)** |
|---|---|---|---|---|---|---|---|
| G1 | M/F | 3/3 | Excipient | IV | - | 0.3 | - |
| G2 | M/F | 3/3 | Mitochondria | IV | 25 | 0.3 | 100 |
| G3 | M/F | 3/3 | Mitochondria | IV | 50 | 0.3 | 200 |
| G4 | M/F | 3/3 | Mitochondria | IV | 100 | 0.3 | 400 |

As shown in Table 5, the G1 group received an excipient. The G2 to G4 groups received mitochondria at 25 µg, 50 µg, or 100 µg, respectively. Here, the G4 group received mitochondria at an amount exceeding the approximate lethal dose (ALD). Here, administration was performed by disinfecting an administration site with 70% alcohol swab, and then administering the excipient or mitochondria via the tail vein at a rate of 1 mL/min using a syringe equipped with a 26-gauge needle. First, for all mice, the type and extent of general symptoms including death, were observed at least once a day during the breeding period and were recorded for each mouse. However, on the day of administration, observation was continued for up to 1 hour after administration, and thereafter, observation was made at 1-hour intervals for 5 hours. Moribund animals and dead animals were treated in accordance with the planned autopsy animals. The start day of administration of the excipient or mitochondria started was set as Day 1.

As a result of observing the general symptoms, no dead animals were observed in all groups during the entire test period; and the abnormal symptoms observed on Day 1 after administration of mitochondria were not observed during the subsequent test period, and thus it is considered to be temporary changes caused by the mitochondria. In addition, body weight was measured for each mouse before administration, and 2 days, 4 days, 8 days, and 15 days after administration. The measurement results are shown in Table 6 below.

**[Table 6]**

| | **Male body weight (g)** | | | | **Female body weight (g)** | | | |
|---|---|---|---|---|---|---|---|---|
| **Day** | **Group** | | | | **Group** | | | |
| | **G1** | **G2** | **G3** | **G4** | **G1** | **G2** | **G3** | **G4** |
| 1 | 37.48±1.78 | 37.88±1.11 | 37.94±1.18 | 38.02±1.07 | 29.12±1.36 | 29.09±1.28 | 29.18±0.99 | 29.32±0.93 |
| 2 | 37.62±1.55 | 38.06±0.55 | 36.46±1.71 | 36.59±1.45 | 29.23±1.48 | 28.96±0.76 | 28.93±0.76 | 28.21±0.96 |
| 4 | 37.50±1.86 | 37.88±0.66 | 36.61±2.17 | 37.46±1.55 | 28.99±0.96 | 28.97±0.61 | 29.54±1.62 | 28.51±1.09 |
| 8 | 38.49±1.53 | 38.75±1.65 | 37.12±3.09 | 38.96±1.79 | 29.13±0.71 | 29.58±0.27 | 29.90±1.78 | 28.92±1.79 |
| 15 | 39.21±1.11 | 39.19±1.17 | 37.73±2.85 | 39.98±1.39 | 30.56±0.52 | 30.22±0.45 | 30.82±1.43 | 29.70±1.39 |
| N | 3 | 3 | 3 | 3 | 3 | 3 | 3 | 3 |

As shown in Table 6, no significant changes in body weight were observed in all G1 to G4 groups. In addition, on Day 15, all mice individuals were anesthetized, and then subjected to laparotomy so as to visually examine all organs. As a result, no changes in organs were observed in all G1 to G4 groups. From the above results, it was found that, when the mitochondria were intravenously administered once to ICR mice under these test conditions, both male and female mice showed no toxicity in a concentration of mitochondria up to 100 µg/head.

### Example 11. Comparison of Characteristics of Mitochondria Derived from Cryopreserved L6 Cells and Mitochondria Derived from Cultured L6 Cells

L6 cells (ATCC, CRL-1458), a myoblast cell line derived from rat skeletal muscle, were inoculated in DMEM-High glucose medium supplemented with 10% fetal bovine serum (FBS, Gibco) and cultured for 72 hours. After the culture was completed, washing was performed twice using DPBS. Then, the washed cells were treated with 0.25% trypsin-EDTA (TE) to obtain cells. The obtained cells were resuspended so as to become 1×10⁷ cells/mL using a hemocytometer. Then, the resultant was placed in a freeze tube, transferred to a cryopreservation vessel, and then frozen at a temperature of -80°C for 24 hours and preserved in a liquid nitrogen cryopreservation tank.

Isolation of mitochondria from the cryopreserved L6 cells was performed in the same manner as in Preparation Example 1, and the thus isolated mitochondria were compared with mitochondria isolated from cultured cells in Preparation Example 1, in terms of ATP activity and membrane potential characteristics. In order to identify normal states of ATP and membrane potential activity of mitochondria, the respective mitochondria were treated with carbonyl cyanide 3-chlorophenylhydrazone (CCCP), which artificially inhibits oxidative phosphorylation, and were used as comparative groups.

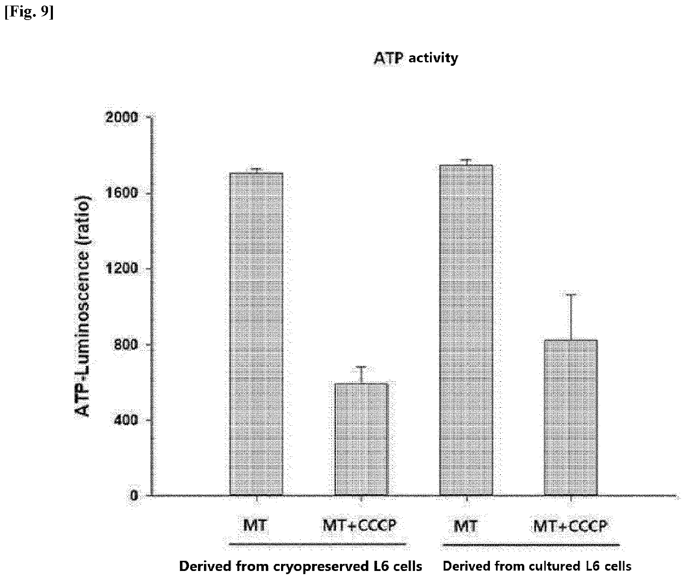

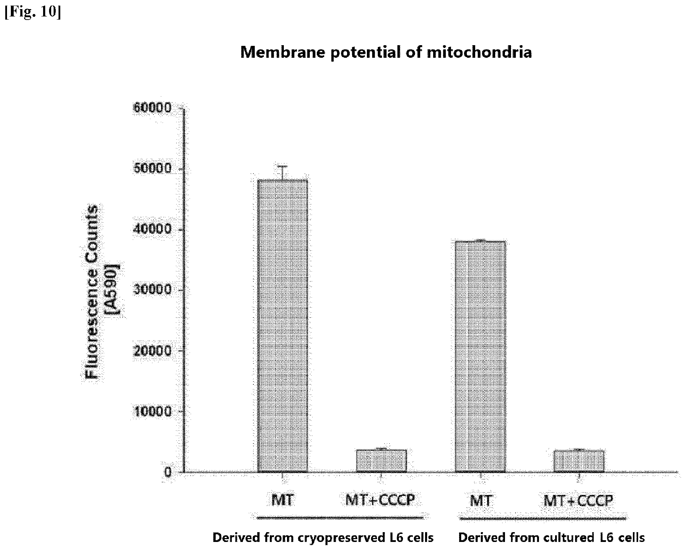

As a result, mitochondria derived from the cryopreserved L6 cells and mitochondria derived from the cultured L6 cells exhibited similar ATP and membrane potential activity. When the respective mitochondria were treated with CCCP, mitochondria derived from the cryopreserved L6 cells exhibited about 65.6% decrease in ATP activity and an about 90% decrease in membrane potential, as compared with the mitochondria not treated with CCCP. In addition, mitochondria derived from the cultured L6 cells exhibited about 48.1% decrease in ATP activity and an about 93% decrease in membrane potential. Thus, it was found, through treatment with CCCP, that the isolated mitochondria exhibited normal ATP and membrane potential activity (Figs. 9 and 10).

### Example 12. Measurement of Number of Mitochondria Using Particle Counter

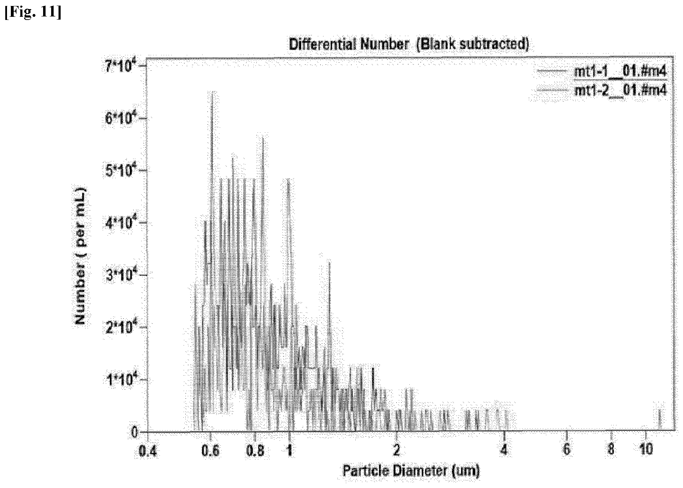

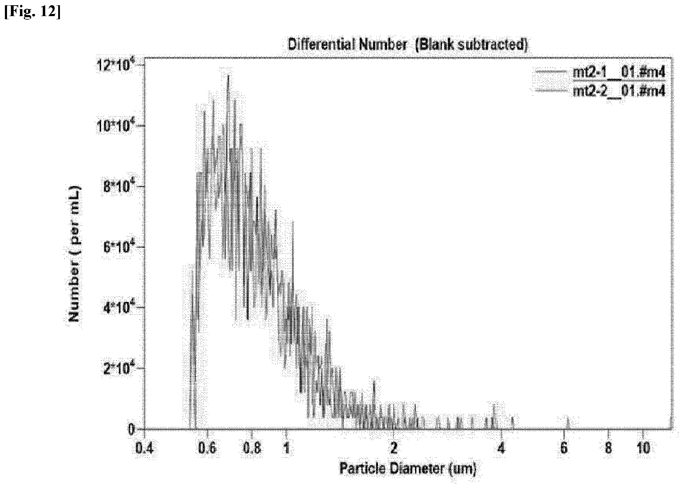

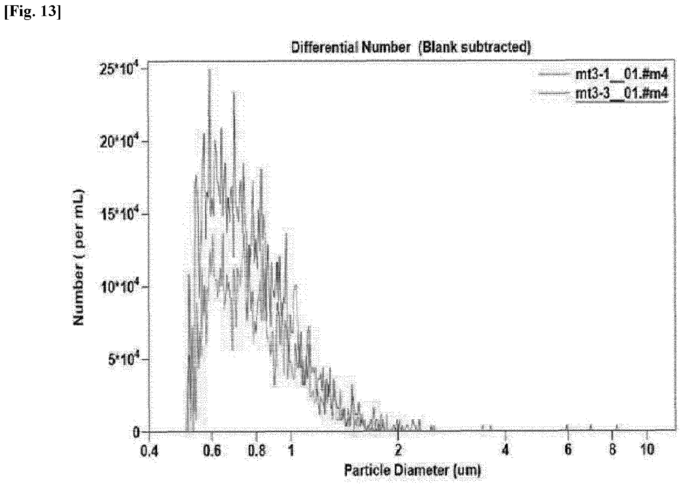

The mitochondria isolated from mesenchymal stem cells derived from human umbilical cord isolated in Preparation Example 2 were used to prepare solutions having mitochondria in a concentration of 1 µg/mL, 2.5 µg/mL, and 5 µg/mL, respectively. Then, a particle counter (Multisizer 4e, Beckman Coulter) was used to measure the number of mitochondria. Here, measurement was performed twice for each concentration, and the measurement results are shown in Table 7 below and Figs. 11 to 13.

**[Table 7]**

| | | | | | | |
|---|---|---|---|---|---|---|
| | 1 µg/mL | | 2.5 µg/mL | | 5 µg/mL | |
| Number of mitochondria /mL | First | Second | First | Second | First | Second |
| | 2.66E±06 | 1.25E±06 | 6.11E±06 | 5.83E±06 | 1.24E±07 | 7.83E±06 |
| Average | 1.96×10⁶ ± 0.98×10⁶ | | 5.97×10⁶ ± 0.19×10⁶ | | 1.01×10⁷ ± 0.32×10⁷ | |

## Claims

1. A pharmaceutical composition for treating sepsis or systemic inflammatory response syndrome (SIRS), comprising mitochondria as an effective ingredient.

2. The pharmaceutical composition of claim 1, wherein the mitochondria are isolated from cells.

3. The pharmaceutical composition of claim 2, wherein the cells are any one selected from the group consisting of somatic cells, germ cells, stem cells, blood cells, and a combination thereof.

4. The pharmaceutical composition of claim 2, wherein the cells are cultured cells or cells stored after culture.

5. The pharmaceutical composition of claim 1, wherein the mitochondria is isolated from platelets.

6. The pharmaceutical composition of claim 3, wherein the stem cells are any one selected from the group consisting of mesenchymal stem cells, induced pluripotent stem cells, embryonic stem cells, and a combination thereof.

7. The pharmaceutical composition of claim 6, wherein the mesenchymal stem cells are obtained from any one selected from the group consisting of umbilical cord, umbilical cord blood, bone marrow, fat, muscle, nerve, skin, amniotic membrane, placenta, synovial fluid, testis, periosteum, and a combination thereof.

8. The pharmaceutical composition of claim 1, wherein the mitochondria in the pharmaceutical composition are included at a concentration of 0.1 µg/mL to 1,000 µg/mL,

9. The pharmaceutical composition of claim 1, wherein the mitochondria are included at a content of 1×10⁵ to 5×10⁹ mitochondria/mL with respect to the pharmaceutical composition.

10. The pharmaceutical composition of claim 1, further comprising an antibiotic or an antiviral agent.

11. The pharmaceutical composition of claim 10, wherein the antibiotic is any one selected from the group consisting of doripenem, cefepime, imipenem, meropenem, ceftazidime, ceftaroline fosamil, ceftriaxone, imipenem, vancomycin, teicoplanin, cefotaxime, ceftazidime, metronidazole, penicillin and aminoglycosides.

12. The pharmaceutical composition of claim 10, wherein the antiviral agent is any one selected from the group consisting of oseltamivir, zanamivir, peramivir, acyclovir, valacyclovir, famciclovir, trifluridine, lamivudine, telbivudine, clevudine, entecavir, adefovir, tenofovir disoproxil, tenofovir alafenamide, besifovir, ribavirin, dasabuvir, sofosbuvir, daclatasvir, asunaprevir, zidovudine, abacavir, efavirenz, etravirine, nevirapine, rilpivirine, atazanavir, darunavir, nelfinavir, ritonavir, indinavir, dolutegravir, raltegravir, enfuvirtide, maraviroc, and a combination thereof.

13. A kit for preventing or treating sepsis or SIRS, comprising a first composition containing mitochondria as an effective ingredient and a second composition containing one or more antibiotics or antiviral agents as an effective ingredient.

14. The use of mitochondria for the treatment of sepsis or SIRS.
